# EUROPÄISCHE PATENTANMELDUNG

(11) **EP 0 618 201 A1**
(43) Veröffentlichungstag der Anmeldung: **05.10.1994**
(21) Anmeldenummer: 94104349.9
(22) Anmeldetag: 19.03.1994
(51) Int. Cl.: C07D 285/16, C07D 417/04, A61K 31/54

(54) **Thiadiazinone und ihre verwendung zur Bekämpfung von Cardiovaskulären sowie asthmatischen erkrankkungen**

(30) Priorität: 01.04.1993 DE 4310699
(71) Anmelder: MERCK PATENT GmbH, D-64293 Darmstadt (DE)
(72) Erfinder: Jonas, Rochus, Dr., D-64291 Darmstadt (DE); Klockow, Michael, Dr., D-64380 Rossdorf (DE); Schliep, Hans-Jochen, Dr., D-64367 Mühltal (DE); Wolf, Michael, Dr., D-64297 Darmstadt (DE)

(57) **Zusammenfassung**

Thiadiazinone der Formel I 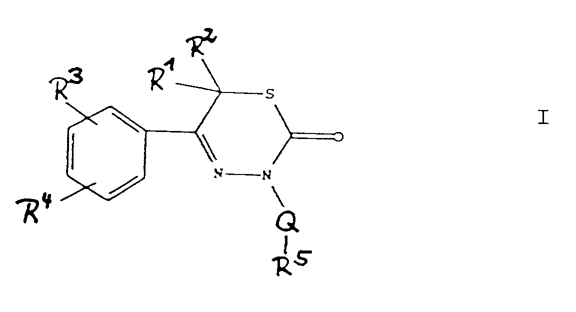
worin Q Alkylen und R⁵ eine gegebenenfalls cyclische Aminogruppe bedeuten, zeigen Phosphodiesterase-hemmende Wirkung und eignen sich zur Bekämpfung von cardiovaskulären sowie asthmatischen Erkrankungen.

## Beschreibung

Die Erfindung betrifft Thiadiazinonderivate der Formel I 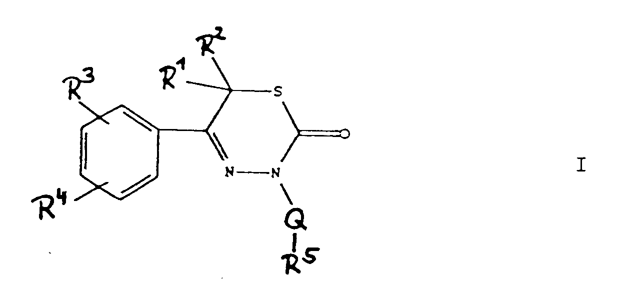
worin
- R¹ und R²: jeweils unabhängig voneinander H oder A,
- R³ und R⁴: jeweils unabhängig voneinander -OH, -OA, -S-A, -SO-A, -SO₂-A, Hal, Methylendioxy, Cycloalkyloxy mit 3-7 C-Atomen oder O-CₘH₂ₘ₊₁₋ₖFₖ,
- R⁵: -NR⁶R⁷ oder 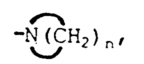 wobei eine CH₂-Gruppe auch durch Sauerstoff ersetzt sein kann,
- R⁶ und R⁷: jeweils unabhängig voneinander H oder A,
- Q: Alkylen mit 1-6 C-Atomen,
- A: Alkyl mit 1-6 C-Atomen,
- Hal: F, Cl, Br oder I,
- m: 1, 2, 3, 4, 5 oder 6,
- n: 3, 4, 5 oder 6
und
- k: 1, 2, 3, 4, 5, 6, 7, 8, 9, 10, 11, 12 oder 13
bedeuten,
sowie deren physiologisch unbedenklichen Salze.

Thiadiazinone sind beispielsweise aus DE 3719031A1 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I wertvolle pharmakologische Eigenschaften besitzen und zudem eine gute Verträglichkeit aufweisen.

Insbesondere zeigen sie eine Phosphodiesterase-Hemmung und können zur Behandlung von asthmatischen Erkrankungen eingesetzt werden. Die antiasthmatische Wirkung kann z.B. nach der Methode von T. Olsson, Acta allergologica 26, 438-447 (1971), bestimmt werden. Ferner zeigen die Verbindungen eine cerebroprotektive Wirkung und haben antidepressive sowie antiphlogistische Eigenschaften.

Sie können zur Behandlung von Gedächtnisstörungen eingesetzt werden, sind positiv inotrop sowie vasodilatierend wirksam. Die Substanzen fördern daher die Durchblutung.

Die vasodilatierende und die Herzwirkung kann z.B. an narkotisierten oder wachen Hunden, Katzen, Affen oder Minischweinen, die positiv inotrope Wirkung auch an isolierten Herz-präparaten (z.B. Vorhof, Papillarmuskel oder perfundiertes Ganzherz) von Ratte, Meerschweinchen, Katze und Hund ermittelt werden, z.B. nach Methoden, wie sie in Arzneimittelforschung, Band 31 (I) Nr. 1a (1981), Seiten 141 bis 170, oder von Schließ et al. im 9th International Congress of Pharmacol., London, Abstracts of papers 9P, beschrieben sind.

Außerdem besitzen die Substanzen antiallergische Eigenschaften.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zu ihrer Herstellung, dadurch gekennzeichnet, daß man eine Verbindung der Formel II, 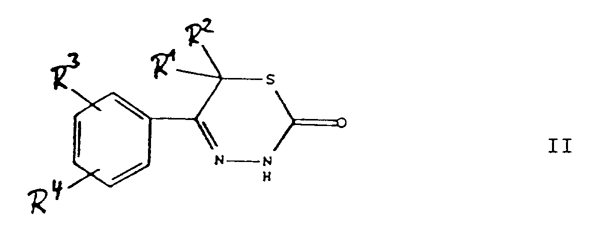
worin R¹, R², R³ und R⁴ die angegebenen Bedeutungen haben mit einer Verbindung der Formel III

R⁵-Q-X III

worin
- R⁵ und Q: die angegebenen Bedeutungen haben und
- X: Cl, Br, OH oder eine reaktionsfähige veresterte OH-Gruppe bedeutet,
umsetzt,
oder daß man eine Verbindung der Formel IV 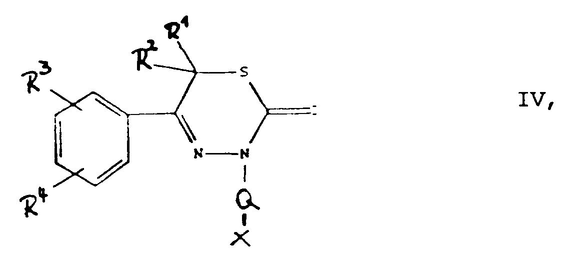
worin
- R¹, R², R³, R⁴, Q und X: die bereits angegebenen Bedeutungen besitzen,
mit einem Amin der Formel V

HNR⁶R⁷ V,

worin
- R⁶ und R⁷: die bereits angegebenen Bedeutungen haben,
oder mit einem Amin der Formel VI, 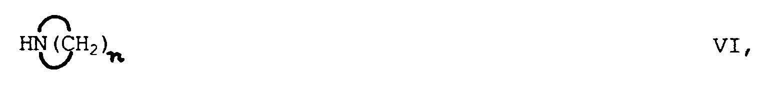
wobei eine CH₂-Gruppe auch durch O ersetzt sein kann und worin
- n: die bereits angegebene Bedeutung besitzt,
umsetzt,
oder daß man eine Verbindung, die der Formel I entspricht, jedoch anstelle von R⁵ eine primäre oder eine sekundäre Aminogruppe besitzt, auf übliche Weise alkyliert,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-X bzw.R⁴-X, worin R³, R⁴ und X die angegebenen Bedeutungen haben, umsetzt und/oder eine Base der Formel I durch Behandeln mit einer Säure in eines ihrer Salze umwandelt.

Vor- und nachstehend haben die Reste R¹, R², R³, R⁴, R⁵, R⁶, R⁷, Q und X sowie die Parameter m, n und k die bei den Formeln I, II, III, IV, V und VI angegebenen Bedeutungen, wenn nicht ausdrücklich etwas anderes angegeben ist.

In den Formeln ist Alkyl vorzugsweise unverzweigt, hat vorzugsweise 1, 2, 3 oder 4 C-Atome und steht bevorzugt für Methyl, ferner bevorzugt für Ethyl oder Propyl, weiterhin bevorzugt für Isopropyl, Butyl, Isobutyl, sek.-Butyl, tert.-Butyl, aber auch für n-Pentyl oder Isopentyl.

Alkoxy ist vorzugsweise unverzweigt, hat vorzugsweise 1, 2 oder 3 C-Atome und steht bevorzugt für Methoxy, ferner bevorzugt für Ethoxy oder Propoxy, weiterhin z.B. für Isopropoxy, Butoxy, Isobutoxy, sek.-Butoxy, tert.-Butoxy, Pentoxy oder Isopentoxy.

Alkylen ist vorzugsweise unverzweigt und bedeutet bevorzugt Methylen oder Butylen, besonders bevorzugt Ethylen oder Propylen.

Von den Resten R¹ und R² steht einer vorzugsweise für H, während der andere bevorzugt Propyl oder Butyl, besonders bevorzugt aber Ethyl oder Methyl bedeutet.

Die Reste R³ und R⁴ können gleich oder verschieden sein und stehen vorzugsweise in der 3- oder 4-Position des Phenylrings. Sie bedeuten beispielsweise unabhängig voneinander Hydroxy, -S-CH₃, -SO-CH₃, -SO₂CH₃, F, Cl, Br oder I oder zusammen Methylendioxy. Besonders bevorzugt stehen sie aber für Methoxy, Ethoxy, Propoxy oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.

Der Rest R⁵ bedeutet vorzugsweise Methylamino, Dimethylamino, Ethylamino, Methylethylamino, Diethylamino oder aber Pyrrolidino, Piperidino oder Morpholino, während m und k vorzugsweise jeweils 1, 2 oder 3 und n vorzugsweise 4 oder 5, wobei eine CH₂-Gruppe durch 0 ersetzt sein kann, bedeuten.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch
in Ia R³ und R⁴ in 3- bzw. 4-Stellung des Phenylrings stehen und
- R¹: H,
- R²: H oder Alkyl,
- R³: OA
bedeuten;
in Ib R³ und R⁴ in 3- bzw. 4-Stellung des Phenylrings stehen und
- R¹: H,
- R²: Methyl oder Ethyl,
- R³ und R⁴: jeweils OA
bedeuten;
in Ic R³ und R⁴ in 3- bzw. 4-Stellung des Phenylrings stehen und
- R¹: H,
- R²: Methyl oder Ethyl,
- R³: OA,
- R⁴: Mono-, Di- oder Trifluoralkoxy
bedeuten;
in Id R³ und R⁴ in 3- bzw. 4-Stellung des Phenylrings stehen und
- R¹: H,
- R²: Methyl oder Ethyl,
- R³: Mono-, Di- oder Trifluoralkoxy,
- R⁴: OA
bedeuten;
in Ie R³ und R⁴ in 3- bzw. 4-Stellung des Phenylrings stehen und
- R¹: H,
- R²: Methyl oder Ethyl,
- R³ und R⁴: jeweils unabhängig voneinander OA oder Mono-, Di- oder Trifluoralkoxy und
- R⁵-Q: 3-Dimethylaminopropyl, 2-Dimethylaminoethyl, 3-Methylaminopropyl, 2-Methylaminoethyl, 3-Ethylaminopropyl oder 2-Ethylaminoethyl
bedeuten;
in If R³ und R⁴ in 3- bzw. 4-Stellung des Phenylrings stehen und
- R¹: H,
- R²: Methyl oder Ethyl,
- R³ und R⁴: jeweils unabhängig voneinander OA oder Mono-, Di- oder Trifluoralkoxy und
- R⁵-Q: Pyrrolidinoethyl, Pyrrolidinopropyl, Piperidinoethyl, Piperidinopropyl, Morpholinoethyl oder Morpholinopropyl bedeuten.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formel II haben R¹, R², R³ und R⁴ die angegebenen Bedeutungen, insbesondere die angegebenen bevorzugten Bedeutungen.

In den Verbindungen der Formel III steht R⁵-Q vorzugsweise für Methylamino-, Dimethylamino-, Ethylamino-, Diethylamino-, Ethylmethylaminopropyl- oder -ethyl bzw. für Pyrrolidino-, Piperidino- oder Morpholinoethyl oder -propyl, während X Cl, Br, OH oder eine reaktionsfähig veresterte OH-Gruppe bedeutet. Falls X eine reaktionsfähige veresterte OH-Gruppe bedeutet, so ist diese vorzugsweise Alkylsulfonyloxy mit 1-6 C-Atomen, z.B. Methansulfonyloxy oder Arylsulfonyloxy mit 6-10 C-Atomen, z.B. Benzol-, p-Toluol- oder 1- oder 2-Naphthalinsulfonyloxy.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die Ausgangsstoffe der Formeln II und III sind teilweise bekannt. Sofern sie nicht bekannt sind, können sie nach an sich bekannten Methoden hergestellt werden.

Thiadiazinone der Formel II und ihre Herstellung sind z.B. in der deutschen Patentanmeldung P 4134893 beschrieben.

Verbindungen der Formel III können beispielsweise durch Umsetzung von geeigneten Dihalogenalkanen mit den entsprechenden primären oder sekundären Aminen hergestellt werden. Weiterhin kann man ausgehend von entsprechenden Aminoalkenen die funktionellen Gruppen X durch anti-Markownikow-Addition von HX an die Doppelbindung herstellen (x = Halogen). Es ist darüber hinaus möglich, ausgehend von den entsprechenden Aminoalkoholen, diese durch an sich bekannte Reaktionen zu verestern, so daß beispielsweise reaktionsfähige Verbindungen mit Alkylsulfonyloxy- oder Arylsulfonyloxygruppen, wie z.B. die entsprechenden Mesylate oder Tosylate entstehen.

Im einzelnen erfolgt die Umsetzung der Thiadiazinone der Formel II mit den Verbindungen der Formel III in Gegenwart oder Abwesenheit eines inerten Lösungsmittels bei Temperaturen zwischen etwa -20 und etwa +150°, vorzugsweise zwischen 20 und 100°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole und Glykolether wie Ethylenglykol, Diethylenglykol, 2-Methoxyethanol; Nitrile wie Acetonitril; Ether wie Tetrahydrofuran oder Dioxan; Amide wie Dimethylformamid (DMF); Sulfoxide wie Dimethylsulfoxid. Auch Gemische dieser Lösungsmittel sind geeignet.

Ferner ist es möglich eine Verbindung der Formel I dadurch herzustellen, daß man eine Verbindung der Formel IV, worin R¹, R², R³, R⁴, Q und X die bereits angegebenen Bedeutungen besitzen, mit einem Amin der Formel V oder VI umsetzt.

Verbindungen der Formel IV können durch Alkylierung von Thiadiazinonen der Formel II nach an sich bekannten und dem Fachmann geläufigen Methoden hergestellt werden, während die Amine der Formeln V und VI in der Regel bekannte Verbindungen darstellen, die im Handel angeboten werden.

Weiterhin kann man eine Verbindung, die der Formel I entspricht, jedoch anstelle von R⁵ eine primäre oder sekundäre Aminogruppe besitzt, auf übliche Weise alkylieren.

Ebenso ist es möglich eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ sowie X die angegebenen Bedeutungen haben, umzusetzen. Die Veretherung der OH-Gruppen erfolgt nach an sich bekannten Methoden, wie sie in Standardwerken der chemischen Literatur (z.B. in Houben-Weyl, Methoden der Organischen Chemie, Georg Thieme Verlag, Stuttgart oder in Organic Reactions, John Wiley & Sons Inc., New York) beschrieben sind, und zwar unter Reaktionsbedingungen, wie sie für die genannten Umsetzungen bekannt und geeignet sind. Dabei kann auch von an sich bekannten, hier nicht näher erwähnten Varianten Gebrauch gemacht werden.

Eine erhaltene Base der Formel I kann mit einer Säure in das zugehörige Säureadditionssalz überführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure,Isonicotinsäure, Methan- oder Ethansulfonsäure, Ethandisulfonsäure, 2-Hydroxyethansulfonsäure, Benzolsulfonsäure, p-Toluolsulfonsäure, Naphthalin- mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Verbindungen der Formel I können eine oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehyrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten, insbesondere von asthmatischen Erkrankungen und von Herzinsuffizienz sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen, wie z.B. Amrinon®, oder Antiasthmatika, wie z.B. Atrovent®, verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":
Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

### Beispiel 1

Eine Lösung von 1,5 g 5-(3,4-Dimethoxyphenyl)-6-ethyl-3,6-dihydro-1,3,4-thiadiazin-2-on [erhältlich durch Umsetzung von 1-(3,4-Dimethoxyphenyl)-2-bromoutan-1-on mit Hydrazinthioameisensäuremethylester] in 30 ml Dimethylformamid (DMF) wird mit 0,6 g K-tert.-butylat versetzt und 30 Minuten gerührt. Anschließend wird eine Lösung von 3-Chlorpropyldimethylamin in Toluol zugegeben und drei Std. gekocht. Man entfernt das Lösungsmittel im Vakuum, arbeitet wie üblich auf und erhält 3-Dimethylaminopropyl-5-(3,4- dimethoxyphenyl)-6-ethyl-3,6-dihydro-1,3,4-thiadiazin-2-on, F. 175° (Hydrochlorid).

Analog erhält man durch Umsetzung von 3-Chlorpropyldimethylamin
- mit: 5-(3-Methoxy-4-trifluormethoxyphenyl)-6-ethyl-3,6-dihydro-1,3,4-thiadiazin-2-on:
3-Dimethylaminopropyl-5-(3-methoxy-4-trifluormethoxyphenyl)-6-ethyl-3,6-dihydro-1,3,4-thiadiazin-2-on;
- mit: 5-(3-Methoxy-4-difluormethoxyphenyl)-6-ethyl-3,6-dihydro-1,3,4-thiadiazin-2-on:
3-Dimethylaminopropyl-5-(3-methoxy-4-difluormethoxyphenyl)-6-ethyl-3,6-dihydro-1,3,4-thiadiazin-2-on;
- mit: 5-(3-Methoxy-4-fluormethoxyphenyl)-6-ethyl-3,6-dihydro-1,3,4-thiadiazin-2-on:
3-Dimethylaminopropyl-5-(3-methoxy-4-fluormethoxyphenyl)-6-ethyl-3,6-dihydro-1,3,4-thiadiazin-2-on;
- mit: 5-(3-Difluormethoxy-4-methoxyphenyl)-6-ethyl-3,6-dihydro-1,3,4-thiadiazin-2-on:
3-Dimethylaminopropyl-5-(3-Difluormethoxy-4-methoxyphenyl)-6-ethyl-3,6-dihydro-1,3,4-thiadiazin-2-on, F. 139° (Fumarat);
- mit: 5-[3-(2,2,2-Trifluorethoxy)-4-methoxyphenyl]-6-ethyl-3,6-dihydro-1,3,4-thiadiazin-2-on:
2-Dimethylaminoethyl-5-[3-(2,2,2-Trifluorethoxy)-4-methoxyphenyl]-6-ethyl-3,6-dihydro-1,3,4-thiadiazin-2-on, F. 155° (Fumarat);
- mit: 5-(3-Fluormethoxy-4-methoxyphenyl)-6-ethyl-3,6-dihydro-1,3,4-thiadiazin-2-on:
3-Dimethylaminopropyl-5-(3-Fluormethoxy-4-methoxyphenyl)-6-ethyl-3,6-dihydro-1,3,4-thiadiazin-2-on;
- mit: 5-(3-Methoxy-4-ethoxyphenyl)-6-ethyl-3,6-dihydro-1,3,4-thiadiazin-2-on:
3-Dimethylaminopropyl-5-(3-methoxy-4-ethoxyphenyl)-6-ethyl-3,6-dihydro-1,3,4-thiadiazin-2-on, F.102° (Fumarat);
- mit: 5-(3-Ethoxy-4-methoxyphenyl)-6-ethyl-3,6-dihydro-1,3,4-thiadiazin-2-on:
3-Dimethylaminopropyl-5-(3-ethoxy-4-methoxyphenyl)-6-ethyl-3,6-dihydro-1,3,4-thiadiazin-2-on, F. 181° (Fumarat);
- mit: 5-(3-Methoxy-4-hydroxyphenyl)-6-ethyl-3,6-dihydro-1,3,4-thiadiazin-2-on:
3-Dimethylaminopropyl-5-(3-methoxy-4-hydroxyphenyl)-6-ethyl-3,6-dihydro-1,3,4-thiadiazin-2-on.

### Beispiel 2

Analog Beispiel 1 erhält man durch Umsetzung von 5-(3,4-Dimethoxyphenyl)-3,6-dihydro-1,3,4-thiadiazin-2-on mit 3-Chlorpropyldimethylamin das 3-Dimethylaminopropyl-5-(3,4-dimethoxyphenyl)-3,6-dihydro-1,3,4-thiadiazin-2-on; F. 195°.

Analog erhält man durch Umsetzung von 2-Chlorethyldimethylamin mit 5-(3,4-Dimethoxyphenyl)-6-ethyl-3,6-dihydro-1,3,4-thiadiazin-2-on:
2-Dimethylaminoethyl-5-(3,4-dimethoxyphenyl)-6-ethyl-3,6-di- hydro-1,3,4-thiadiazin-2-on;
- mit: 5-(3-Methoxy-4-trifluormethoxyphenyl)-6-ethyl-3,6-dihydro-1,3,4-thiadiazin-2-on:
2-Dimethylaminoethyl-5-(3-methoxy-4-trifluormethoxyphenyl)-6-ethyl-3,6-dihydro-1,3,4-thiadiazin-2-on;
- mit: 5-(3-Methoxy-4-difluormethoxyphenyl)-6-ethyl-3,6-dihydro-1,3,4-thiadiazin-2-on:
2-Dimethylaminoethyl-5-(3-methoxy-4-difluormethoxyphenyl)-6-ethyl-3,6-dihydro-1,3,4-thiadiazin-2-on;
- mit: 5-(3-Methoxy-4-fluormethoxyphenyl)-6-ethyl-3,6-dihydro-1,3,4-thiadiazin-2-on:
2-Dimethylaminoethyl-5-(3-methoxy-4-fluormethoxyphenyl)-6-ethyl-3,6-dihydro-1,3,4-thiadiazin-2-on;
- mit: 5-(3-Difluormethoxy-4-methoxyphenyl)-6-ethyl-3,6-dihydro-1,3,4-thiadiazin-2-on:
2-Dimethylaminoethyl-5-(3-Difluormethoxy-4-methoxyphenyl)-6-ethyl-3,6-dihydro-1,3,4-thiadiazin-2-on;
- mit: 5-(3-Fluormethoxy-4-methoxyphenyl)-6-ethyl-3,6-dihydro-1,3,4-thiadiazin-2-on:
2-Dimethylaminoethyl-5-(3-Fluormethoxy-4-methoxyphenyl)-6-ethyl-3,6-dihydro-1,3,4-thiadiazin-2-on;
- mit: 5-(3-Methoxy-4-ethoxyphenyl)-6-ethyl-3,6-dihydro-1,3,4-thiadiazin-2-on:
2-Dimethylaminoethyl-5-(3-methoxy-4-ethoxyphenyl)-6-ethyl-3,6-dihydro-1,3,4-thiadiazin-2-on;
- mit: 5-(3-Ethoxy-4-methoxyphenyl)-6-ethyl-3,6-dihydro-1,3,4-thiadiazin-2-on:
2-Dimethylaminoethyl-5-(3-ethoxy-4-methoxyphenyl)-6-ethyl-3,6-dihydro-1,3,4-thiadiazin-2-on;
- mit: 5-(3-Hydroxy-4-methoxyphenyl)-6-ethyl-3,6-dihydro-1,3,4-thiadiazin-2-on:
2-Dimethylaminoethyl-5-(3-hydroxy-4-methoxyphenyl)-6-ethyl-3,6-dihydro-1,3,4-thiadiazin-2-on.

### Beispiel 3

Eine Lösung von 2,3 g 3-Morpholinopropyl-5-(3-methoxy-4-hydroxyphenyl)-6-ethyl-3,6-dihydro-1,3,4-thiadiazin-2-on [erhältlich durch Umsetzung von 1-(4-Hydroxy-3-methoxyphenyl)-2-brom-butan-1-on mit Hydrazinthioameisensäuremethylester und nachfolgende Reaktion mit 1-Chlor-3-morpholinopropan] in THF wird nach Zugabe von einem Äquivalent 3-Iod-1,1,2,2,3-pentafluorpropan zwei Std. gekocht. Anschließend wird das Lösungsmittel im Vakuum entfernt und wie üblich aufgearbeitet. Man erhält 3-Morpholinopropyl-5-[3-methoxy-4-(1,1,2,2,3-pentafluorpropoxy-phenyl)]-6-ethyl-3,6-dihydro-1,3,4-thiadiazin-2-on.

Analog erhält man durch Veretherung der entsprechenden Mono- oder Dihydroxy-phenyl-1,3,4-thiadiazinonderivate mit Polyfluoralkylhalogeniden:
3-Dimethylaminopropyl-5-(3-methoxy-4-trifluormethoxyphenyl)-3,6-dihydro-1,3,4-thiadiazin-2-on;
3-Dimethylaminopropyl-5-(4-trifluormethoxyphenyl)-3,6-dihydro-1,3,4-thiadiazin-2-on;
3-Dimethylaminopropyl-5-[3,4-bis-(difluormethoxy)-phenyl]-3,6-dihydro-1,3,4-thiadiazin-2-on;
3-Dimethylaminopropyl-5-[3-methoxy-4-(1,1,2-trifluorethoxy)phenyl]-3,6-dihydro-1,3,4-thiadiazin-2-on;
3-Dimethylaminopropyl-5-[3,4-bis-(chlormethoxy)-phenyl]-3,6-dihydro-1,3,4-thiadiazin-2-on.

### Beispiel 4

Analog Beispiel 1 erhält man durch Umsetzung von 5-(3-Methoxy-4-fluormethoxyphenyl)-6-ethyl-3,6-dihydro-1,3,4-thiadiazin-2-on mit 1-Chlor-3-morpholinopropan das 3-Morpholinopropyl-5-(3-methoxy-4-fluormethoxyphenyl)-6-ethyl-3,6-dihydro-1,3,4-thiadiazin-2-on.

Analog erhält man durch Umsetzung
- von: 5-(3-Methoxy-4-trifluormethoxyphenyl)-6-ethyl-3,6-dihydro-1,3,4-thiadiazin-2-on mit 1-Chlor-3-morpholinopropan:
3-Morpholinopropyl-5-(3-methoxy-4-trifluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-3-piperidinopropan:
3-Piperidinopropyl-5-(3-methoxy-4-difluormethoxyphenyl)-6-ethyl-3,6-dihydro-1,3,4-thiadiazin-2-on;
- von: 5-(3,4-Dimethoxyphenyl)-6-ethyl-3,6-dihydro-1,3,4-thiadiazin-2-on mit 1-Chlor-3-morpholinopropan:
3-Morpholinopropyl-5-(3,4-dimethoyyphenyl)-6-ethyl-3,6-dihydro-1,3,4-thiadiazin-2-on, F. 136 ° (Oxalat);
- von: 5-(3,4-Dimethoxyphenyl)-6-ethyl-3,6-dihydro-1,3,4-thiadiazin-2-on mit 1-Chlor-3-piperidinopropan:
3-Piperidinopropyl-5-(3,4-dimethoxyphenyl)-6-ethyl-3,6- dihydro-1,3,4-thiadiazin-2-on, F.121°, (Oxalat);
- von: 5-(3,4-Dimethoxyphenyl)-6-ethyl-3,6-dihydro-1,3,4-thiadiazin-2-on mit 1-Chlor-3-pyrrolidinopropan:
3-Pyrrolidinopropyl-5-(3,4-dimethoxyphenyl)-6-ethyl-3,6-dihydro-1,3,4-thiadiazin-2-on;
- von: 5-(3-Methoxy-4-ethoxyphenyl)-6-ethyl-3,6-dihydro-1,3,4-thiadiazin-2-on mit 1-Chlor-3-morpholinopropan:
3-Morpholinopropyl-5-(3-methoxy-4-ethoxyphenyl)-6-ethyl-3,6-dihydro-1,3,4-thiadiazin-2-on, F. 157° (Fumarat);
- von: 5-(3-Methoxy-4-ethoxyphenyl)-6-ethyl-3,6-dihydro-1,3,4-thiadiazin-2-on mit 1-Chlor-3-piperidinopropan:
3-Piperidinopropyl-5-(3-methoxy-4-ethoxyphenyl)-6-ethyl-3,6-dihydro-1,3,4-thiadiazin-2-on, F. 134° (Fumarat);
- von: 5-(3-Methoxy-4-ethoxyphenyl)-6-ethyl-3,6-dihydro-1,3,4-thiadiazin-2-on mit 1-Chlor-3-pyrrolidinopropan:
3-Pyrrolidinopropyl-5-(3-methoxy-4-ethoxyphenyl)-6-ethyl-3,6-dihydro-1,3,4-thiadiazin-2-on;
- von: 5-(3-Ethoxy-4-methoxyphenyl)-6-ethyl-3,6-dihydro-1,3,4-thiadiazin-2-on mit 1-Chlor-3-morpholinopropan:
3-Morpholinopropyl-5-(3-Ethoxy-4-methoxyphenyl)-6-ethyl-3,6-dihydro-1,3,4-thiadiazin-2-on, F. 190° (Fumarat);
- von: 5-(3-Ethoxy-4-methoxyphenyl)-6-ethyl-3,6-dihydro-1,3,4-thiadiazin-2-on mit 1-Chlor-3-piperidinopropan:
3-Piperidinopropyl-5-(3-Ethoxy-4-methoxyphenyl)-6-ethyl-3,6-dihydro-1,3,4-thiadiazin-2-on, F. 165° (Fumarat);
- von: 5-(3-Methoxy-4-difluormethoxyphenyl)-6-ethyl-3,6-dihydro-1,3,4-thiadiazin-2-on mit 1-Chlor-3-morpholinopropan:
3-Morpholinopropyl-5-(3-methoxy-4-difluormethoxyphenyl)-6-ethyl-3,6-dihydro-1,3,4-thiadiazin-2-on, F. 118° (Fumarat);
- von: 5-(3-Difluormethoxy-4-methoxyphenyl)-6-ethyl-3,6-dihydro-1,3,4-thiadiazin-2-on mit 1-Chlor-3-piperidinopropan:
3-Piperidinopropyl-5-(3-Difluormethoxy-4-methoxyphenyl)-6-ethyl-3,6-dihydro-1,3,4-thiadiazin-2-on, F. 104° (Hydrochlorid);
- von: 5-(3-Methoxy-4-difluormethoxyphenyl)-6-ethyl-3,6-dihydro-1,3,4-thiadiazin-2-on mit 1-Chlor-3-pyrrolidinopropan:
3-Pyrrolidinopropyl-5-(3-methoxy-4-difluormethoxyphenyl)-6-ethyl-3,6-dihydro-1,3,4-thiadiazin-2-on;
- von: 5-(3-Methoxy-4-fluormethoxyphenyl)-6-ethyl-3,6-dihydro-1,3,4-thiadiazin-2-on mit 1-Chlor-3-morpholinopropan:
3-Morpholinopropyl-5-(3-methoxy-4-fluormethoxyphenyl)-6-ethyl-3,6-dihydro-1,3,4-thiadiazin-2-on;
- von: 5-(3-Methoxy-4-fluormethoxyphenyl)-6-ethyl-3,6-dihydro-1,3,4-thiadiazin-2-on mit 1-Chlor-3-piperidinopropan:
3-Piperidinopropyl-5-(3-methoxy-4-fluormethoxyphenyl)-6-ethyl-3,6-dihydro-1,3,4-thiadiazin-2-on;
- von: 5-(3-Methoxy-4-fluormethoxyphenyl)-6-ethyl-3,6-dihydro-1,3,4-thiadiazin-2-on mit 1-Chlor-3-pyrrolidinopropan:
3-Pyrrolidinopropyl-5-(3-methoxy-4-fluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-3-morpholinopropan:
3-Morpholinopropyl-5-(3-methoxy-4-difluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-3-piperidinopropan:
3-Piperidinopropyl-5-(3-methoxy-4-difluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-3-pyrrolidinopropan:
3-Pyrrolidinopropyl-5-(3-methoxy-4-difluormethoxyphenyl)-6-ethyl-3,6-dihydro-1,3,4-thiadiazin-2-on;
- von: 5-[3-(2,2,2-Trifluorethoxy)-4-methoxyphenyl]-6-ethyl-3,6-dihydro-1,3,4-thiadiazin-2-on mit 1-Chlor-3-piperidinopropan:
3-Piperidinopropyl-5-[3-(2,2,2-Trifluorethoxy)-4-methoxyphenyl]-6-ethyl-3,6-dihydro-1,3,4-thiadiazin-2-on, F. 142° (Fumarat);
- von: 5-[3-(2,2,2-Trifluorethoxy)-4-methoxyphenyl]-6-ethyl-3,6-dihydro-1,3,4-thiadiazin-2-on mit 1-Chlor-3-morpholinopropan:
3-Morpholinopropyl-5-[3-(2,2,2-Trifluorethoxy)-4-methoxyphenyl]-6-ethyl-3,6-dihydro-1,3,4-thiadiazin-2-on, F. 162° (Fumarat).

### Beispiel 5

Analog Beispiel 4 erhält man durch Umsetzung von 5-(3-Methoxy-4-fluormethoxyphenyl)-6-ethyl-3,6-dihydro-1,3,4-thiadiazin-2-on mit 1-Chlor-2-morpholinoethan das 2-Morpholinoethyl-5-(3-methoxy-4-fluormethoxyphenyl)-6-ethyl-3,6-dihydro-1,3,4-thiadiazin-2-on.

Analog erhält man durch Umsetzung
- von: 5-(3-Methoxy-4-trifluormethoxyphenyl)-6-ethyl-3,6-dihydro-1,3,4-thiadiazin-2-on mit 1-Chlor-2-morpholinoethan:
2-Morpholinoethyl-5-(3-methoxy-4-trifluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-piperidinoethan:
2-Piperidinoethyl-5-(3-methoxy-4-difluormethoxyphenyl)-6-ethyl-3,6-dihydro-1,3,4-thiadiazin-2-on;
- von: 5-(3,4-Dimethoxyphenyl)-6-ethyl-3,6-dihydro-1,3,4-thiadiazin-2-on mit 1-Chlor-2-morpholinoethan:
2-Morpholinoethyl-5-(3,4-dimethoxyphenyl)-6-ethyl-3,6-dihydro-1,3,4-thiadiazin-2-on;
- von: 5-(3,4-Dimethoxyphenyl)-6-ethyl-3,6-dihydro-1,3,4-thiadiazin-2-on mit 1-Chlor-2-piperidinoethan:
2-Piperidinoethyl-5-(3,4-dimethoxyphenyl)-6-ethyl-3,6-dihydro-1,3,4-thiadiazin-2-on;
- von: 5-(3,4-Dimethoxyphenyl)-6-ethyl-3,6-dihydro-1,3,4-thiadiazin-2-on mit 1-Chlor-2-pyrrolidinoethan:
2-Pyrrolidinoethyl-5-(3,4-dimethoxyphenyl)-6-ethyl-3,6-dihydro-1,3,4-thiadiazin-2-on;
- von: 5-(3-Methoxy-4-ethoxyphenyl)-6-ethyl-3,6-dihydro-1,3,4-thiadiazin-2-on mit 1-Chlor-2-morpholinoethan:
2-Morpholinoethyl-5-(3-methoxy-4-ethoxyphenyl)-6-ethyl-3,6-dihydro-1,3,4-thiadiazin-2-on;
- von: 5-(3-Methoxy-4-ethoxyphenyl)-6-ethyl-3,6-dihydro-1,3,4-thiadiazin-2-on mit 1-Chlor-2-piperidinoethan:
2-Piperidinoethyl-5-(3-methoxy-4-ethoxyphenyl)-6-ethyl-3,6-dihydro-1,3,4-thiadiazin-2-on;
- von: 5-(3-Methoxy-4-ethoxyphenyl)-6-ethyl-3,6-dihydro-1,3,4-thiadiazin-2-on mit 1-Chlor-2-pyrrolidinoethan:
2-Pyrrolidinoethyl-5-(3-methoxy-4-ethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-morpholinoethan:
2-Morpholinoethyl-5-(3-methoxy-4-difluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-piperidinoethan:
2-Piperidinoethyl-5-(3-methoxy-4-difluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-pyrrolidinoethan:
2-Pyrrolidinoethyl-5-(3-methoxy-4-difluormethoxyphenyl)-6-ethyl-3,6-dihydro-1,3,4-thiadiazin-2-on;
- von: 5-(3-Methoxy-4-fluormethoxyphenyl)-6-ethyl-3,6-dihydro-1,3,4-thiadiazin-2-on mit 1-Chlor-2-morpholinoethan:
2-Morpholinoethyl-5-(3-methoxy-4-fluormethoxyphenyl)-6-ethyl-3,6-dihydro-1,3,4-thiadiazin-2-on;
- von: 5-(3-Methoxy-4-fluormethoxyphenyl)-6-ethyl-3,6-dihydro-1,3,4-thiadiazin-2-on mit 1-Chlor-2-piperidinoethan:
2-Piperidinoethyl-5-(3-methoxy-4-fluormethoxyphenyl)-6-ethyl-3,6-dihydro-1,3,4-thiadiazin-2-on;
- von: 5-(3-Methoxy-4-fluormethoxyphenyl)-6-ethyl-3,6-dihydro-1,3,4-thiadiazin-2-on mit 1-Chlor-2-pyrrolidinoethan:
2-Pyrrolidinoethyl-5-(3-methoxy-4-fluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-morpholinoethan:
2-Morpholinoethyl-5-(3-methoxy-4-difluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-piperidinoethan:
2-Piperidinoethyl-5-(3-methoxy-4-difluormethoxyphenyl)-6-ethyl-3,6-dihydro-1,3,4-thiadiazin-2-on;
- von: 5-(3-Methoxy-4-difluormethoxyphenyl)-6-ethyl-3,6-dihydro-1,3,4-thiadiazin-2-on mit 1-Chlor-2-pyrrolidinoethan:
2-Pyrrolidinoethyl-5-(3-methoxy-4-difluormethoxyphenyl)-6-ethyl-3,6-dihydro-1,3,4-thiadiazin-2-on.

Die nachstehenden Beispiele betreffen pharmazeutische Zubereitungen.

### Beispiel A: Injektionsgläser

Eine Lösung von 100 g 3-Dimethylaminopropyl-5-(3,4-dimethoxyphenyl)-6-ethyl-3,6-dihydro-1,3,4-thiadiazinon und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g 3-Dimethylaminopropyl-5-(3,4-dimethoxyphenyl)-3,6-dihydro-1,3,4-thiadiazin-on mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g 3-Piperidinopropyl-5-(3-methoxy-4-difluormethoxyphenyl)-6-ethyl-3,6-dihydro-1,3,4-thiadiazin-2-on, 9,38 g NaH₂PO₄ x 2 H₂O, 28,48 g Na₂HPO₄ x 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg 3-Morpholinopropyl-5-(3-methoxy-4-difluormethoxyphenyl)-6-ethyl-3,6-dihydro-1,3,4-thiadiazin-2-on mit 99,5 g Vaseline unter aseptischen Bedingungen.

### Beispiel E: Tabletten

Ein Gemisch von 1 kg 3-Pyrrolidinopropyl-5-(3-methoxy-4-fluormethoxyphenyl)-6-ethyl-3,6-dihydro-1,3,4-thiadiazin-2-on, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg 3-Pyrrolidinopropyl-5-(3-methoxy-4-fluormethoxyphenyl)-6-ethyl-3,6-dihydro-1,3,4-thiadiazin-2-on werden in üblicher Weise in Hartgelatinekapseln gefüllt, so daß jede Kapsel 20 mg des Wirkstoffs enthält.

### Beispiel H: Ampullen

Eine Lösung von 1 kg 3-Pyrrolidinopropyl-5-(3-methoxy-4-fluormethoxyphenyl)-6-ethyl-3,6-dihydro-1,3,4-thiadiazin-2-on in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind alle genannten pharmazeutischen Zubereitungen erhältlich, die einen oder mehrere der übrigen Wirkstoffe der Formel I und/oder ihre physiologisch unbedenklichen Säureadditionssalze enthalten.

## Patentansprüche

1. Thiadiazinonderivate der Formel I 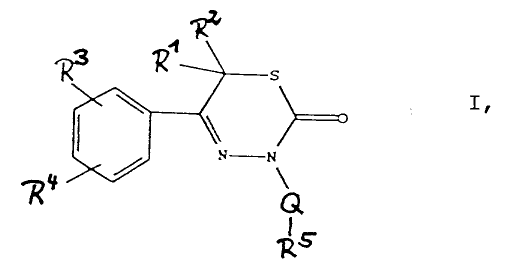 worin
R¹ und R² jeweils unabhängig voneinander H oder A,
R³ und R⁴ jeweils unabhängig voneinander -OH, -OA, -S-A, -SO-A, -SO₂-A, Hal, Methylendioxy, Cycloalkyloxy mit 3-7 C-Atomen oder O-CₘH₂ₘ₊₁₋ₖFₖ,
R⁵ -NR⁶R⁷ oder 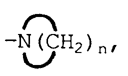 wobei eine CH₂-Gruppe auch durch Sauerstoff ersetzt sein kann,
R⁶ und R⁷ jeweils unabhängig voneinander H oder A,
Q Alkylen mit 1-6 C-Atomen,
A Alkyl mit 1-6 C-Atomen,
Hal F, Cl, Br oder I,
m 1, 2, 3, 4, 5 oder 6,
n 3, 4, 5 oder 6
und
k 1, 2, 3, 4, 5, 6, 7, 8, 9, 10, 11, 12 oder 13
bedeuten,
sowie deren physiologisch unbedenklichen Salze.

2. Ein reines Enantiomer einer Verbindung der Formel I gemäß Anspruch 1.

3. a) 3-Dimethylamino-propyl-5-(3,4-dimethoxyphenyl)-6-ethyl-3,6-dihydro-1,3,4-thiadiazin-2-on;
b) 3-Dimethylamino-propyl-5-(3,4-dimethoxyphenyl)-3,6-dihydro-1,3,4-thiadiazin-2-on;
c) 3-Dimethylamino-ethyl-5-(3,4-dimethoxyphenyl)-6-ethyl-3,6-dihydro-1,3,4-thiadiazin-2-on;
d) 3-Piperidinopropyl-5-(3-methoxy-4-difluormethoxyphenyl)-6-ethyl-3,6-dihydro-1,3,4-thiadiazin-2-on;
e) 3-Morpholinopropyl-5-(3-methoxy-4-difluormethoxyphenyl)-6-ethyl-3,6-dihydro-1,3,4-thiadiazin-2-on.

4. Verfahren zur Herstellung von Verbindungen der Formel I nach Anspruch 1 sowie von deren Salzen, dadurch gekennzeichnet, daß man eine Verbindung der Formel II 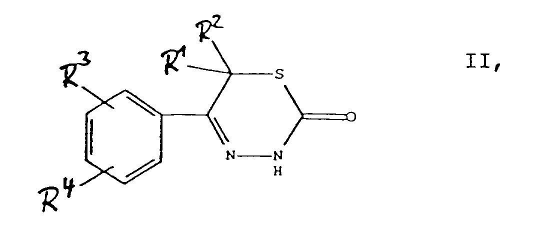 worin
R¹, R², R³ und R⁴ die bereits angegebenen Bedeutungen haben,
mit einer Verbindung der Formel III
R⁵-Q-X III,
worin
R⁵ und Q die angegebenen Bedeutungen haben und
X Cl, Br, OH oder eine reaktionsfähige veresterte OH-Gruppe bedeutet,
umsetzt,
oder daß man eine Verbindung der Formel IV 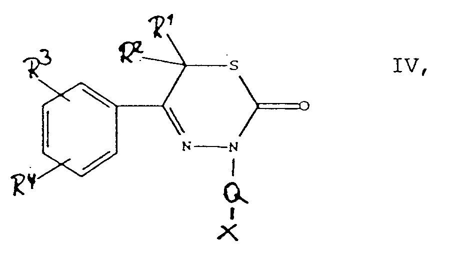 worin
R¹, R², R³, R⁴, Q und X die bereits angegebenen Bedeutungen besitzen,
mit einem Amin der Formel V
HNR⁶R⁷ V,
worin
R⁶ und R⁷ die bereits angegebenen Bedeutungen haben,
oder mit einem Amin der Formel VI 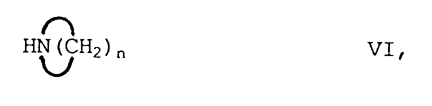 wobei auch eine CH₂-Gruppe durch Sauerstoff ersetzt sein kann und
worin
n die bereits angegebene Bedeutung besitzt,
umsetzt,
oder daß man eine Verbindung, die der Formel I entspricht, jedoch anstelle von R⁵ eine primäre oder eine sekundäre Aminogruppe besitzt, auf übliche Weise alkyliert,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ und X die angegebenen Bedeutungen haben, umsetzt und/oder eine Base der Formel I durch Behandeln mit einer Säure in eines ihrer Salze umwandelt.

5. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der Formel I nach Anspruch 1 und/oder einem ihrer physiologisch unbedenklichen Salze.

7. Verwendung einer Verbindung der Formel I nach Anspruch 1 und/oder eines ihrer physiologisch unbedenklichen Salze zur Herstellung eines Arzneimittels zur Bekämpfung von Krankheiten.

8. Verwendung von Verbindungen der Formel I und/oder ihrer physiologisch unbedenklichen Salze bei der Bekämpfung von Krankheiten.
